# FASCICULE DE BREVET EUROPEEN

(11) **EP 1 263 361 B1**
(45) Date de publication et mention de la délivrance du brevet: **06.04.2005**
(21) Numéro de dépôt: 01917154.5
(22) Date de dépôt: 15.03.2001
(51) Int. Cl.: A61F 6/18

(54) **DISPOSITIF D'INSERTION POUR LA MISE EN PLACE D'UN DISPOSITIF INTRA-UTERIN DU TYPE EN FORME DE T**
VORRICHTUNG ZUM EINFÜHREN EINER T-FÖRMIGEN INTRAUTERINEN VORRICHTUNG
DEVICE FOR INSERTING A T-SHAPED INTRA-UTERINE DEVICE

(30) Priorité: 16.03.2000 FR 0003405
(43) Date de publication de la demande: 11.12.2002
(73) Titulaire: Solinhac, Jean-Pierre, 33740 Ares (FR)
(72) Inventeur: Solinhac, Jean-Pierre, 33740 Ares (FR)
(74) Mandataire: Thébault, Jean-Louis
(86) Numéro de dépôt international: PCT/FR2001/000780
(87) Numéro de publication internationale: WO 2001/068012

(56) Documents cités:
- US-A- 3 918 444
- US-A- 3 918 445
- US-A- 4 143 656
- US-A- 4 361 150

## Description

La présente invention a trait à un dispositif d'insertion pour la mise en place d'un dispositif intra-utérin du type en forme de T.

Un tel dispositif, plus communément appelé stérilet, est un dispositif contraceptif constitué d'un corps principal de forme tubulaire définissant par exemple un réservoir contenant un progestatif entouré d'une membrane de contrôle de diffusion, prolongé à une extrémité par deux ailes diamétralement opposées, l'ensemble ayant la forme d'un T, et muni à son autre extrémité d'un fil permettant l'extraction du dispositif hors de l'utérus.

On connaît déjà divers dispositifs d'insertion destinés à la mise en place correcte dans l'utérus de ce type de stérilet.

Par US 4 143 656, on connaît un dispositif d'insertion prêt à l'emploi comportant un conduit dans lequel est conservé jusqu'à son utilisation le stérilet en T dans sa position déployée, les deux ailes faisant saillie hors dudit conduit par deux lumières opposées ménagées dans le conduit.

Lors de l'insertion du stérilet, une tige-piston pré-engagée dans le conduit pousse le stérilet en sorte, en un premier temps, de contraindre les ailes du stérilet à rentrer dans le conduit en se plaquant contre le corps du stérilet, en un second temps, de propulser le stérilet dans cet état jusqu'à l'extrémité distale du conduit, puis, en un troisième temps, de faire sortir le stérilet de façon à ce qu'il déploie ses ailes.

Ce dispositif s'adresse essentiellement à des stérilets en T conçus pour que les ailes soient rabattables contre le corps du stérilet et est particulièrement complexe puisque le conduit d'insertion est en deux parties aboutées, respectivement proximale et distale, totalement différentes.

La partie proximale, qui est celle comportant lesdites lumières, est en matériau rigide et de section ovale afin de recevoir à l'intérieur le stérilet avec ses ailes rabattues contre le corps de celui-ci. La partie distale, dans le prolongement de la partie proximale, est de section circulaire et en matériau souple afin de se déformer pour laisser passer le stérilet poussé par la tige-poussoir.

Par ailleurs, la partie distale est munie d'une bague coulissante de forme ovale, coopérant avec une graduation ménagée sur la face externe de la partie distale et indiquant la profondeur d'engagement de cette dernière dans la cavité utérine.

Un tel ensemble est de fabrication délicate et coûteuse, peu pratique d'emploi et est inutilisable pour l'insertion de stérilet en T notamment en matériau à mémoire de forme, conçu pour un repliage des ailes l'une contre l'autre mais dans le prolongement dudit stérilet.

Dans d'autres dispositifs d'insertion, destinés précisément à des stérilets en T à ailes repliables l'une contre l'autre dans le prolongement du corps du stérilet, le stérilet est stocké, avant utilisation, dans un tube d'insertion, dans sa position de déploiement desdites ailes, seul le corps du stérilet étant engagé dans la partie distale dudit tube, une tige-piston étant pré-engagée dans le tube d'insertion.

Au moment de la mise en place du stérilet, l'ensemble du stérilet est rétracté à l'intérieur du tube par traction sur le fil d'extraction du stérilet dépassant à l'autre extrémité du tube, ce dernier est introduit dans l'utérus, puis la tige-piston est poussée pour extraire et libérer en place le stérilet.

Quel que soit le type de dispositif, il est nécessaire d'introduire le tube d'insertion dans l'utérus. Une telle introduction dans l'utérus et plus particulièrement le franchissement du col est toujours délicate et pose quelquefois de réelles difficultés.

Du fait que le tube d'insertion enveloppe totalement le stérilet et doive à son extrémité distale laisser totalement passer le stérilet pour son déploiement, ladite extrémité distale doit avoir un diamètre suffisant, adapté à celui du stérilet. Le tube doit par ailleurs présenter une rigidité suffisante pour que l'ensemble puisse franchir le col de l'utérus, normalement fermé.

L'extrémité de l'ensemble n'étant donc pas arrondie et de surcroît présentant, dans le cas du dispositif selon US 4 143 656, une section élargie pour laisser passer le stérilet avec ses ailes repliées contre son corps, il s'ensuit une difficulté plus ou moins grande pour forcer le passage du col de l'utérus, accompagnée inévitablement d'un inconfort pour la patiente, pouvant être aggravé quelquefois par la survenue de malaise, de perte de connaissance, de risque de lésion. Parfois, la pose est impossible sans dilatation préalable du col nécessitant souvent une anesthésie locale non dénuée de risque pour la patiente.

La présente invention a précisément pour but de pallier ces inconvénients en proposant un dispositif d'insertion spécifiquement conçu pour une mise en place plus facile, plus douce et non traumatisante pour la patiente, de dispositifs intro-utérins du type en T à ailes rabattables uniquement dans le prolongement du corps du dispositif intra-utérin, à l'aide d'un dispositif prêt à l'emploi dans lequel le dispositif intra-utérin est stocké avant son utilisation à l'état déployé dans un conduit d'insertion, l'ensemble étant de préférence dans un conditionnement stérile.

A cet effet, l'invention définie par la revendication 1 a pour objet un dispositif d'insertion pour la mise en place d'un dispositif intra-utérin du type en forme de T et à ailes repliables dans le prolongement du corps du dispositif intra-utérin, comprenant un conduit dans lequel est susceptible de coulisser librement ledit dispositif intra-utérin avec ses deux ailes repliées, une bague susceptible de coulisser librement sur ledit conduit et une tige formant piston susceptible de coulisser à l'intérieur dudit conduit, ledit dispositif intra-utérin étant stocké, avant utilisation, dans ledit conduit, à l'état déployé, les ailes faisant saillie hors du conduit par deux lumières diamétralement opposées ménagées dans le conduit, caractérisé en ce que ledit conduit est constitué d'un tube monobloc et de section interne circulaire d'une extrémité à l'autre et comporte, à son extrémité distale, une partie tronconique de diamètre allant en se réduisant, fendue en sorte de définir au moins deux pétales en regard susceptibles de s'écarter élastiquement et en ce que ladite bague coulissante est susceptible d'être amenée en regard desdites lumières en sorte, lors de la mise en place du dispositif intra-utérin et après rétraction des ailes de ce dernier à l'intérieur dudit tube, d'obturer lesdites lumières au passage du dispositif intra-utérin lors de son poussage par ladite tige-piston en direction de ladite extrémité distale dudit tube.

Un tel dispositif d'insertion est de conception simple, facile d'emploi et non traumatisant pour la patiente.

Il est de préférence stocké avant emploi en milieu stérile à l'intérieur d'une poche, le stérilet étant au moment du conditionnement du dispositif et après introduction avec son fil d'extraction à l'intérieur du tube, déployé par amenée des ailes en regard des lumières du tube et conservé dans cet état jusqu'au moment de son utilisation.

Au moment de l'utilisation, une traction sur le fil permet d'escamoter les ailes du stérilet complètement à l'intérieur du tube d'insertion.

Ensuite, la tige-piston est insérée par l'extrémité proximale du tube, les lumières sont obturées à l'aide de la bague coulissante et le stérilet est poussé vers l'extrémité distale du tube.

Le déplacement du stérilet est arrêté avant que ce dernier ne commence à écarter les pétales de l'extrémité distale du tube qui est alors prêt à être introduit dans l'utérus de la patiente.

L'extrémité tronconique arrondie et de diamètre réduit du tube assure une pénétration facile et en douceur dans le col de l'utérus.

Une fois le tube convenablement introduit dans l'utérus, une nouvelle poussée sur la tige-piston provoque l'écartement des pétales antagonistes de l'extrémité distale du tube, le déploiement des ailes du stérilet et la sortie complète de ce dernier, après quoi les pétales reviennent élastiquement dans leur position d'origine et l'ensemble tige-piston peut être retiré en dégageant le fil d'extraction à la manière habituelle.

D'autres caractéristiques et avantages ressortiront de la description qui va suivre d'un mode de réalisation du dispositif d'insertion selon l'invention, description donnée à titre d'exemple uniquement et en regard du dessin annexé sur lequel :
- la figure 1 est une vue en élévation latérale d'un tube d'insertion selon l'invention ;
- la figure 2 est une vue similaire d'une tige-piston selon l'invention, appropriée au tube de la figure 1 ;
- la figure 3 est une vue de l'ensemble formé par les éléments des figures 1 et 2 montés, le tube d'insertion étant vu en coupe et muni d'une bague coulissante ;
- la figure 4 est une vue partielle agrandie de l'extrémité distale du tube d'insertion ;
- la figure 5 est une vue partielle en coupe axiale agrandie de la partie centrale de l'ensemble de la figure 3, montrant le stérilet en place après conditionnement du dispositif et avant utilisation de ce dernier, et
- la figure 6 est une vue analogue à la figure 4 illustrant l'ouverture des pétales de l'extrémité distale du tube d'insertion pour le déploiement du stérilet.

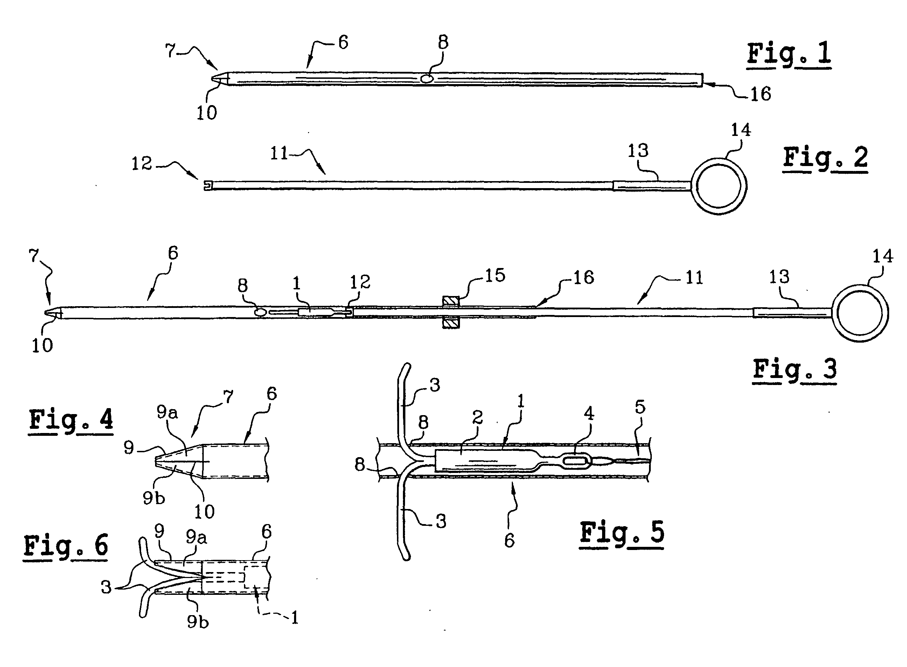

Sur la figure 5, on a représenté un dispositif intra-utérin ou stérilet 1, en forme générale de T à l'état déployé, c'est-à-dire en position d'usage. Plus précisément le stérilet 1 est du type dénommé commercialement Mirena et comprend un corps principal cylindrique allongé 2 constituant un réservoir contenant un progestatif entouré d'une membrane de contrôle de diffusion, deux ailes 3 diamétralement opposées à une extrémité du corps 2, en matière plastique souple à mémoire de forme, susceptibles d'être rapprochées dans le prolongement du corps 2 et de retrouver automatiquement lorsqu'elles sont libérées leur position en alignement orthogonalement audit corps 2.

A l'autre extrémité du corps 2, ce dernier se prolonge par un anneau 4 de fixation d'un fil 5 d'extraction du stérilet hors de l'utérus.

Le dispositif d'insertion du stérilet 1 selon l'invention comprend un tube 6 en matière plastique appropriée à la fois souple mais suffisamment rigide pour permettre son introduction dans l'utérus, notamment son passage dans le col de l'utérus. La section interne du tube est circulaire d'un bout à l'autre du tube.

Le tube 6 est (figure 1) analogue à ceux communément utilisés pour la mise en place de stérilets tels que le stérilet 1 Mirena, mais s'en distingue conformément à l'invention sur deux points essentiels, à savoir l'extrémité distale 7 qui présente une forme générale tronconique et la zone centrale où sont ménagés dans la paroi du tube 6 deux lumières identiques oblongues 8, diamétralement opposées et dont le grand axe est parallèle à l'axe du tube 6.

Le diamètre interne du tube 6 est déterminé de façon à permettre un libre coulissement du stérilet 1 introduit à l'intérieur avec ses ailes 3 repliées l'une contre l'autre dans le prolongement du corps 2.

L'extrémité distale 7 du tube 6 définit (figure 4) une partie tronconique 9 de conicité par exemple de l'ordre de 30° et d'une longueur d'environ 7 mm pour un diamètre externe du tube 6 de 4,6 mm, l'extrémité de la partie 9 étant ouverte et présentant un diamètre de l'ordre de 1,4 mm.

Ces dimensions peuvent bien entendu varier légèrement.

La pointe extrême, c'est-à-dire le bord externe, de la partie 9 peut avantageusement être arrondie.

Par ailleurs, la partie 9 est, dans le mode de réalisation représenté, incisé dans un plan axial au tube 6, suivant deux fentes 10 diamétralement opposées s'étendant depuis la pointe de la partie 9 jusqu'au début de la partie cylindrique du tube 6.

Les fentes 10 définissent ainsi deux parties 9a, 9b en forme approximative de pétales antagonistes susceptibles de s'écarter élastiquement l'une de l'autre comme illustré par la figure 6 pour permettre le passage des ailes 3 du stérilet 1 en vue de leur déploiement comme on le verra plus en détail ultérieurement.

A l'intérieur du tube 6 est susceptible de coulisser une tige-piston 11 (figure 2) en matière plastique, conventionnelle à l'exception de son extrémité distale munie, conformément à l'invention, d'un moyen 12 de préhension de l'extrémité proximale du stérilet 1 à l'intérieur du tube 6, c'est-à-dire de la partie annulaire 4.

Ce moyen 12 constitué par exemple par une sorte de petite fourche est susceptible de s'engager à cheval sur l'extrémité de ladite partie annulaire 4 en vue de permettre la rotation du stérilet 1 à l'intérieur du tube 6 à l'aide de la tige-piston 11, comme on le verra également plus loin.

A son extrémité proximale, la tige-piston 11 comporte à la manière connue une partie 13 de diamètre élargie dont l'extrémité sert de repère d'introduction de la tige 11 à l'intérieur du tube 6, ainsi qu'un anneau terminal de préhension 14 qui fait également office de butée d'enfoncement de la tige 11 à l'intérieur du tube 6, la partie élargie 13 présentant néanmoins un diamètre permettant son introduction dans le tube 6.

On a représenté enfin en 15 sur la figure 3 une bague coulissante montée sur le tube 6 et qui sert habituellement de repère de position.

La partie tronconique 9 du tube 6 est avantageusement réalisée en introduisant l'extrémité du tube 6 dans une empreinte de moule concave tronconique chauffée par induction pour assurer une bonne maîtrise de la température nécessaire pour ramollir la matière du tube 6 et déformer son extrémité à la manière désirée en lui conférant une forme finale permanente bien que déformable élastiquement. Tout autre moyen permettant d'obtenir le même résultat pourrait bien entendu être mis en oeuvre.

Le dispositif de l'invention est conditionné en usine sous poche stérile, le tube 6 et la tige-poussoir 11 étant disposés côté à côte et le stérilet 1 étant inséré dans le tube 6 dans la position déployée illustrée par la figure 5, c'est-à-dire les ailes 3 dépassant hors du tube 6 à travers les lumières 8, les brins de fil 5 sortant du tube 6 par l'extrémité proximale de ce dernier, ces brins n'étant pas représentés sur la figure 3.

Lors du conditionnement, le stérilet 1 avec ses ailes 3 repliées dans le prolongement du corps 2 est introduit dans le tube 6 par son extrémité proximale 16, puis poussé par la tige 11 à l'intérieur du tube 6 en direction des lumières 8. L'extrémité en fourche 12 de la tige-piston 11 permet de saisir l'extrémité annulaire 4 du stérilet 1 et de faire pivoter éventuellement ce dernier à l'intérieur du tube 6 pour présenter les extrémités des ailes 3 en regard des lumières 8 de déploiement.

Une fois les extrémités des ailes 3 arrivées en regard des lumières 8, lesdites extrémités se déploient élastiquement au travers des lumières 8 et sortent jusqu'à complète extension des ailes 3 hors du tube 6 comme illustré par la figure 5. Le stérilet 1 est alors en position normale et va y demeurer à l'intérieur de la poche d'emballage stérile.

Lors de l'utilisation du dispositif, les deux éléments, à savoir le tube 6 avec son stérilet 1 et la bague 15 et la tige-piston 11 sont extraits de la poche stérile. Une traction est exercée sur le fil 5 à l'extrémité proximale du tube 6 pour rétracter totalement les ailes 3 à l'intérieur du tube 6. Ensuite, la bague 15 est amenée à hauteur des lumières 8 pour les occulter, puis la tige-piston 11 est introduite dans l'extrémité proximale du tube 6 et le stérilet 1 est poussé jusqu'à l'extrémité distale du tube 6, sans toutefois forcer l'écartement des pétales 9a, 9b.

Le dispositif est alors prêt à être introduit dans l'utérus de la patiente.

L'extrémité distale épointée 7 du tube 6 facilite le bon positionnement et le passage en douceur du col de l'utérus. Le tube 6 est engagé à la bonne profondeur, c'est à dire au contact du fond utérin.

La bague 15 sert non seulement à éviter que les ailes 3 ne ressortent du tube 6 lorsqu'elles vont repasser à hauteur des lumières 8, mais également à éviter tout contact corporel avec le stérilet lors de sa mise en place.

La mise en place habituelle du stérilet consiste alors à ressortir l'ensemble d'environ 1 cm, puis, en maintenant le stérilet grâce à la tige 11, à rétracter le tube 6 seul jusqu'à ce que l'extrémité proximale 16 du tube 6 vienne à hauteur du repère constitué par l'extrémité de la partie 13 de la tige 11.

Pendant cette opération, les extrémités des ailes 3 du stérilet 1 forcent (figure 6) les deux pétales 9a, 9b à s'écarter permettant aux deux ailes 3 de se déployer, le corps 2 du stérilet demeurant inséré dans l'extrémité du tube 6.

On pousse alors l'ensemble au contact du fond utérin, on amène alors l'extrémité proximale 16 du tube au contact de l'anneau 14, ce qui libère complètement le stérilet.

Une fois le stérilet 1 sorti, les pétales 9a, 9b se "referment" automatiquement grâce à l'élasticité du matériau du tube 6. Ce matériau, ainsi que celui des autres composants du dispositif d'insertion est de préférence du polyéthylène basse densité qualité extrusion tube médical.

L'ensemble tube 6-tige 11 est ensuite retiré, laissant libre le fil 5 dont les extrémités sont coupées à bonne longueur, à la manière habituelle.

Il est à noter que la longueur de fil 5 équipant chaque stérilet 1 peut être, grâce à l'invention, réduite par exemple d'une bonne quinzaine de centimètres du fait que le stérilet est stocké dans la zone centrale du tube 6 et non plus à l'extrémité distale comme dans les dispositifs conventionnels, les extrémités des brins du fil 5 dépassant nécessairement de l'extrémité proximale dudit tube 6.

Enfin, l'invention n'est évidemment pas limitée au mode de réalisation représenté et décrit ci-dessus, mais en couvre au contraire toutes les variantes, notamment en ce qui concerne les dimensions de la partie distale tronconique 7, la forme, le nombre et la longueur des incisions 10, le nombre et la forme des pétales 9a, 9b qui peuvent être supérieurs à deux, les forme et dimensions des lumières 8.

## Revendications

1. Dispositif d'insertion pour la mise en place d'un dispositif intra-utérin (1) du type en forme de T et à ailes repliables dans le prolongement du corps (2) du dispositif intra-utérin, comprenant un conduit (6) dans lequel est susceptible de coulisser librement ledit dispositif intra-utérin (1) avec ses deux ailes (3) repliées, une bague (15) susceptible de coulisser librement sur ledit conduit (6) et une tige formant piston (11) susceptible de coulisser à l'intérieur dudit conduit (6), ledit dispositif intra-utérin étant stocké, avant utilisation, dans ledit conduit (6), à l'état déployé, les ailes (3) faisant saillie hors du conduit par deux lumières (8) diamétralement opposées ménagées dans le conduit, **caractérisé en ce que** ledit conduit est constitué d'un tube monobloc (6) et de section interne circulaire d'une extrémité à l'autre et comporte, à son extrémité distale (7), une partie tronconique (9) de diamètre allant en se réduisant, fendue en sorte de définir au moins deux pétales en regard (9a, 9b) susceptibles de s'écarter élastiquement et **en ce que** ladite bague coulissante (15) est susceptible d'être amenée en regard desdites lumières (8) en sorte, lors de la mise en place du dispositif intra-utérin et après rétraction des ailes de ce dernier à l'intérieur dudit tube (6), d'obturer lesdites lumières au passage du dispositif intra-utérin lors de son poussage par ladite tige-piston en direction de ladite extrémité distale (7) dudit tube.

2. Dispositif suivant la revendication 1, **caractérisé en ce que** les deux pétales (9a, 9b) sont délimités à l'aide d'incisions (10) ménagées dans un plan contenant l'axe du tube (6) sur au moins toute la longueur de ladite partie tronconique (9).

3. Dispositif suivant la revendication 1 ou 2, **caractérisé en ce que** l'extrémité de ladite partie tronconique (9) est arrondie.

4. Dispositif suivant l'une des revendications 1 à 3, **caractérisé en ce que** l'extrémité distale de la tige-piston (11) comporte un moyen (12) de saisie de l'extrémité proximale du stérilet (1) en position à l'intérieur du tube (6), afin de faire pivoter éventuellement le stérilet autour de son axe dans ledit tube.

5. Dispositif suivant l'une des revendications 1 à 4, **caractérisé en ce que** la tige-piston (11) est munie à sa partie proximale, d'une part, d'une partie (13) de diamètre élargi faisant office de repère par rapport à l'extrémité proximale 16 du tube (6), ladite partie (13) étant néanmoins susceptible d'être introduite à l'intérieur dudit tube (6), et, d'autre part, d'un anneau terminal de préhension (14) faisant office de butée d'enfoncement de la tige-piston (11) dans le tube (6).

6. Dispositif suivant l'une des revendications 1 à 5, **caractérisé en ce que** ladite partie tronconique (9) du tube (6) est réalisée à l'aide d'une empreinte de moule tronconique chauffée par induction.

## Patentansprüche

1. Einführvorrichtung für die Anordnung einer intrauterinen Vorrichtung (1) des Typs in T-Form mit Flügeln, die in der Verlängerung des Körpers (2) der intrauterinen Vorrichtung anklappbar sind, mit einem Kanal (6), in dem die intrauterine Vorrichtung (1) mit ihren zwei angeklappten Flügeln (3) frei gleiten kann, einem Ring (15), der auf dem Kanal (6) frei gleiten kann, und einem einen Kolben (11) bildenden Stift, der innerhalb des Kanals (6) gleiten kann, wobei die intrauterine Vorrichtung vor der Verwendung ausgeklappt im Kanal (6) untergebracht ist, wobei die Flügel (3) durch zwei diametral entgegengesetzte Langlöcher (8), die in dem Kanal ausgespart sind, von dem Kanal vorstehen, **dadurch gekennzeichnet, dass** der Kanal aus einem einteiligen Rohr (6) mit kreisförmigem Innenquerschnitt von einem Ende zum anderen gebildet ist und an seinem distalen Ende (7) einen kegelstumpfförmigen Abschnitt (9) mit abnehmendem Durchmesser aufweist, der geschlitzt ist, um wenigstens zwei gegenüberliegende Ansätze (9a, 9b) zu definieren, die sich elastisch voneinander entfernen können, und dass der Gleitring (15) gegenüber den Langlöchern (8) angeordnet werden kann, derart, dass bei der Anordnung der intrauterinen Vorrichtung und nach der Retraktion ihrer Flügel in das Rohr (6) die Langlöcher für den Durchgang der intrauterinen Vorrichtung verschlossen werden, wenn sie durch den Stiftkolben in Richtung des distalen Endes (7) des Rohrs verschoben wird.

2. Vorrichtung nach Anspruch 1, **dadurch gekennzeichnet, dass** die zwei Ansätze (9a, 9b) mittels Einschnitten (10) begrenzt sind, die in einer Ebene, die die Achse des Rohrs (6) enthält, zumindest über die gesamte Länge des kegelstumpfförmigen Teils (9) ausgebildet sind.

3. Vorrichtung nach Anspruch 1 oder 2, **dadurch gekennzeichnet, dass** das Ende des kegelstumpfförmigen Teils (9) abgerundet ist.

4. Vorrichtung nach einem der Ansprüche 1 bis 3, **dadurch gekennzeichnet, dass** das distale Ende des Stiftkolbens (11) ein Mittel (12) aufweist, das vom proximalen Ende der Spirale (1) in der Position innerhalb des Rohrs (6) vorsteht, um die Spirale eventuell um ihre Achse im Rohr zu schwenken.

5. Vorrichtung nach einem der Ansprüche 1 bis 4, **dadurch gekennzeichnet, dass** der Stiftkolben (11) in seinem proximalen Abschnitt einerseits mit einem Abschnitt (13) mit erweitertem Durchmesser, der als Markierung in Bezug auf das proximale Ende (16) des Rohrs (6) dient und dennoch in den Innenraum des Rohrs (6) eingeführt werden kann, und andererseits mit einem Greif-Endring (14), der als Anschlag für die Einführung des Stiftkolbens (11) in das Rohr (6) dient, versehen ist.

6. Vorrichtung nach einem der Ansprüche 1 bis 5, **dadurch gekennzeichnet, dass** der kegelstumpfförmige Abschnitt (9) des Rohrs mit Hilfe einer kegelstumpfförmigen Gießform, die durch Induktion erhitzt wird, verwirklicht ist.

## Claims

1. An insertion device for fitting an intra-uterine device (1) of the T-shaped type and with wings that can be folded in line with the body (2) of the intra-uterine device, comprising a conduit (6) in which said intra-uterine device (1) is able to slide freely with its two wings (3) folded, a ring (15) able to slide freely on said conduit (6) and a rod forming a piston (11) able to slide inside said conduit (6), said intra-uterine device being stored, before use, in said conduit (6), in the deployed state, the wings (3) projecting out of the conduit through two diametrically opposed apertures (8) provided in the conduit, **characterised in that** said conduit is formed from a single-piece tube (6) with a circular internal section from one end to the other and comprises, as its distal end (7), a frustoconical part (9) with a reducing diameter, split so as to define at least two facing petals (9a, 9b) able to move apart elastically, and **in that** said sliding ring (15) is able to be brought opposite said apertures (8) so as, when the intra-uterine device is fitted and after retraction of the wings of the latter inside said tube (6), to close off said apertures as the intra-uterine device passes when it is pushed by said piston rod in the direction of said distal end (7) of said tube.

2. A device according to claim 1, **characterised in that** the two petals (9a, 9b) are delimited by means of incisions (10) provided in a plane containing the axis of the tube (6) over at least the entire length of said frustoconical part (9).

3. A device according to claim 1 or 2, **characterised in that** the end of said frustoconical part (9) is rounded.

4. A device according to one of claims 1 to 3, **characterised in that** the distal end of the piston (11) comprises a means (12) of gripping the proximal end of the IUD (1) in position inside the tube (6), if necessary to make the IUD pivot about its axis in said tube.

5. A device according to one of claims 1 to 4, **characterised in that** the piston rod (11) is provided at its proximal part firstly with a part (13) having an enlarged diameter serving as a reference with respect to the proximal end (16) of the tube (6), said part (13) nevertheless being able to be introduced inside said tube (6), and, secondly, with an end gripping ring (14) serving as a driving-stop for the piston rod (11) in the tube (6).

6. A device according to one of clams 1 to 5 **characterised in that** said frustoconical part (9) of the tube (6) is produced by means of a frustoconical mould cavity heated by induction.
